# EUROPEAN PATENT APPLICATION

(11) **EP 4 421 039 A1**
(43) Date of publication of application: **28.08.2024**
(21) Application number: 22882943.8
(22) Date of filing: 20.10.2022
(51) Int. Cl.: C01B 39/54, C01B 37/08

(54) **SCM-38 MOLECULAR SIEVE, PREPARATION METHOD THEREFOR, AND USE THEREOF**

(30) Priority: 20.10.2021 CN 202111224534; 20.10.2021 CN 202111222065
(71) Applicant: China Petroleum & Chemical Corporation, Beijing 100728 (CN); Shanghai Research Institute of Petrochemical Technology, SINOPEC, Shanghai 201208 (CN)
(72) Inventor: YANG, Weimin, Shanghai 201208 (CN); SHAO, Shengli, Shanghai 201208 (CN); QIAO, Jian, Shanghai 201208 (CN); YUAN, Zhiqing, Shanghai 201208 (CN); WANG, Zhendong, Shanghai 201208 (CN); FU, Wenhua, Shanghai 201208 (CN); TAO, Weichuan, Shanghai 201208 (CN)
(74) Representative: Hoffmann Eitle
(86) International application number: PCT/CN2022/126429
(87) International publication number: WO 2023/066338

(57) **Abstract**

The present invention discloses a SCM-38 molecular sieve and preparation process and use thereof. The SCM-38 molecular sieve is a phosphorus-aluminium or silicon-phosphorus-aluminium molecular sieve, the SCM-38 molecular sieve shows the following diffraction peak feature in its XRD spectrum: X-ray diffraction peaks appear at 2θ of 7.20±0.1, 10.81±0.1, 11.60±0.1, 14.32±0.1, 21.39±0.1, 21.83±0.1, 27.31±0.1, 28.72±0.1, wherein the most intense peak appears at 2θ of 7.20±0.1. The SCM-38 molecular sieve of the present invention can be used in catalysts.

## Description

### Technical Field

The present invention belongs to the field of molecular sieves, and specifically relates to a new molecular sieve, namely SCM-38 molecular sieve, preparation process and use thereof.

### Background of Art

Molecular sieves are a type of porous crystalline materials that are widely used in chemical industries such as oil refining and catalysis. Molecular sieves with different pore structures reflect different macroscopic properties such as adsorption and catalysis. In addition, molecular sieves with different structures have also been synthesized. More than 250 types of molecular sieves with known structures (including locally disordered ones) are currently discovered. Since the molecular sieve has uniform and regular channels, and the channel size is of the same order as that of small organic molecules, the molecules entering the molecular sieve can be "sieved" according to the spatial size of the molecules during chemical reactions, thereby obtaining a certain degree of selective adsorption and catalytic shape-selective effects. The framework of molecular sieves is usually composed of coordination tetrahedrons (TO₄) connected through common vertices (usually oxygen atoms). For conventional zeolite molecular sieves, the tetrahedrons in the framework are mainly silicon-oxygen tetrahedrons and aluminium-oxygen tetrahedrons. These two tetrahedrons can also be replaced by other tetrahedrons respectively, thereby forming various framework structures or molecular sieves composed of various frameworks.

In 1971, Flanigen et al. (Molecular Sieve Zeolites-I, ACS, Washingtom D.C) reported the synthesis of phosphorus-aluminium molecular sieve, which can be understood as the silicon-oxygen tetrahedron in the zeolite molecular sieve was replaced by the phosphorus-oxygen tetrahedron to form a molecular sieve. The framework of this type of molecular sieve is connected by common oxygen atoms of AlO₄⁻and PO₄⁺, and the entire molecular sieve framework is electrically neutral. Similar to zeolite molecular sieves, the aluminium-oxygen tetrahedron or phosphorus-oxygen tetrahedron in the phosphorus-aluminium molecular sieve can also be replaced by other tetrahedrons, the most common of which are silicon-oxygen tetrahedron and zinc-oxygen tetrahedron. Due to the introduction of these tetrahedrons, new characteristics of the phosphorus-aluminium molecular sieve are provided. Compared with zeolite molecular sieves, the artificial synthesis of phosphorus-aluminium molecular sieves was studied relatively late. Under hydrothermal synthesis conditions, the oxides of aluminium, silicon and phosphorus are mixed to obtain a silicon-phosphorus-aluminium molecular sieve with the same crystal structure as those of analcime, chabazite, phillipsite-harmotome, L-type molecular sieve, A-type molecular sieve, B-type molecular sieve and the like, wherein the phosphorus content is 5% to 25% (as P₂O₅), but no molecular sieves with structures different from known zeolite molecular sieves have been found. According to the US patent US 4310440 (1982), organic amines or quaternary ammonium compounds were used as template agents to hydrothermally synthesize a series of phosphorus-aluminium molecular sieves, including AlPO₄-5, AlPO₄-8, AlPO₄-9, AlPO₄-11, AlPO₄-12, AlPO₄-14, AlPO₄-16, AlPO₄-17, AlPO₄-18, AlPO₄-20, AlPO₄-21, AlPO₄-22, AlPO₄-23, AlPO₄-25, AlPO₄-26, AlPO₄-28, AlPO₄-31 and the like. With the deepening understanding of molecular sieve structure, properties, synthesis methods, conditions and other factors and the continuous advancement of synthesis technology, molecular sieves with new structures are continuously being synthesized. For synthesizing phosphorus-aluminium molecular sieves, the type of organic template agent is one of the key factors determining their structures. So far, organic amines are still the most widely used template agents in the synthesis of phosphorus-aluminium molecular sieves. Compared with silicon-aluminium zeolite molecular sieves, phosphorus-aluminium molecular sieves are rarely used in industrial applications. Currently, only a few molecular sieves such as SAPO-34 and SAPO-11 molecular sieves have been used in practical industrial applications. Jiao et. al (Feng Jiao, Jinjing Li, Xiulian Pan, et al. Science, 2016, 351, 1065-1068) reported that SAPO molecular sieve was used as a part of the coupling catalyst in the reaction of producing olefins from the synthesis gas with achieving good catalytic effects. Su et al. (Su, J., Zhou, H., Liu, S. et al. Syngas to light olefins conversion with high olefin/paraffin ratio using ZnCrOx/AlPO-18 bifunctional catalysts. Nat Commun 10, 1297 (2019).) disclosed that a bifunctional catalyst prepared from a phosphorus-aluminium molecular sieve and a metal oxide has excellent performance in the method of directly converting synthesis gas to produce light olefins with a high olefin/alkane ratio. From the above, it can be seen that the phosphorus-aluminium molecular sieve has great industrial application potential.

Since different channel structures and elemental compositions determine the unique physical, chemical and catalytic properties of molecular sieves, the development of new structural molecular sieves is particularly important.

### Summary of the Invention

The technical problem to be solved by the present invention is to provide a new type of molecular sieve (phosphorus-aluminium molecular sieve or silicon-phosphorus-aluminium molecular sieve) not involved in the prior art, namely SCM-38 molecular sieve, preparation process and use thereof.

A first aspect of the present invention provides a SCM-38 molecular sieve, wherein the molecular sieve is a phosphorus-aluminium or silicon-phosphorus-aluminium molecular sieve, the SCM-38 molecular sieve shows the following diffraction peak feature in its XRD spectrum: X-ray diffraction peaks appear at 2θ diffraction angles of 7.20±0.1, 10.81±0.1, 11.60±0.1, 14.32±0.1, 21.39±0.1, 21.83±0.1, 27.31±0.1, 28.72±0.1, wherein the most intense peak appears at 2θ of 7.20±0.1,
the SCM-38 molecular sieve has a schematic chemical composition represented by "P₂O₅·Al₂O₃·SiO₂", wherein based on the molar ratio, Al₂O₃:P₂O₅=0.8-1.5:1, preferably 0.8-1.4, more preferably 0.9-1.3; SiO₂:P₂O₅=0-0.1:1, preferably 0-0.09:1, more preferably 0-0.07:1.

A second aspect of the present invention provides a process for preparing the above-mentioned SCM-38 molecular sieve, comprising the following steps:
a) mixing an aluminophosphate precursor, a quaternary ammonium compound 1, a quaternary ammonium compound 2, a fluorine source and water as well as an optional aluminium source, and an optional silicon source to produce a synthesis mother liquor;
b) crystallizing the synthesis mother liquor obtained in step a) to produce a SCM-38 molecular sieve, wherein, the quaternary ammonium compound 1 is a compound represented by the following formula (I), 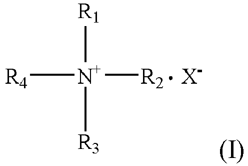
   wherein, R₁, R₂, R₃ and R₄ are each independently selected from C₁-C₆ linear or branched alkyl (preferably C₁-C₄ linear or branched alkyl, more preferably C₁-C₃ linear or branched alkyl), X⁻represents a counter ion (preferably halogen anion, nitrate group, hydroxyl group),
   the quaternary ammonium compound 2 is a compound represented by the following formula (II), 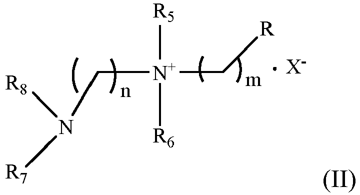
   wherein R₅, R₆, R₇ and R₈ are each independently selected from C₁-C₆ linear or branched alkyl (preferably C₁-C₄ linear or branched alkyl, more preferably C₁-C₃ linear or branched alkyl), R represents hydrogen or hydroxy, X⁻ represents a counter ion (preferably halogen anion, nitrate group, hydroxyl group), m represents an integral of 1-4 (preferably an integral of 1-3, more preferably an integral of 1-2), n represents an integral of 5-10 (preferably an integral of 5-8, more preferably an integral of 5-6),
   the counter ion X⁻ in at least one of the compound represented by formula (I) and the compound represented by formula (II) represents hydroxyl group.

A third aspect of the present invention provides a molecular sieve composition, containing the above-mentioned SCM-38 molecular sieve of the present invention and a binder.

A fourth aspect of the present invention provides a catalyst for producing dimethyl ether from methanol, containing the above-mentioned SCM-38 molecular sieve of the present invention or the above-mentioned molecular sieve composition of the present invention.

A fifth aspect of the present invention provides a method for producing dimethyl ether from methanol, which method comprises a step of converting methanol to dimethyl ether in the presence of a catalyst, wherein the catalyst contains the above-mentioned SCM-38 molecular sieve of the present invention or the above-mentioned molecular sieve composition of the present invention.

### Technical effect

The present invention provides a new phosphorus-aluminium molecular sieve or silicon-phosphorus-aluminium molecular sieve.

The phosphorus-aluminium molecular sieve or the silicon-phosphor-aluminium molecular sieve of the present invention exhibits excellent activity as a catalyst for converting methanol into dimethyl ether. The process for preparing the molecular sieve of the present invention can prepare the molecular sieve of the present invention conveniently and efficiently.

### Description of the drawings

Figure 1 is the XRD spectrum of the aluminophosphate precursor A obtained in Example 1;
Figure 2 is the XRD spectrum of the aluminophosphate precursor B obtained in Example 2;
Figure 3 is the XRD spectrum of the SCM-38 molecular sieve obtained in Example 3;
Figure 4 is the SEM image of the SCM-38 molecular sieve obtained in Example 3;
Figure 5 is the XRD spectrum of the SCM-38 molecular sieve obtained in Example 10;
Figure 6 is the SEM image of the SCM-38 molecular sieve obtained in Example 10;
Figure 7 is the XRD spectrum of the SCM-38 molecular sieve obtained in Example 13;
Figure 8 is the SEM image of the SCM-38 molecular sieve obtained in Example 13;
Figure 9 is the XRD spectrum of the product obtained in Comparative Example 1;
Figure 10 is the XRD spectrum of the product obtained in Comparative Example 2;
Figure 11 is the XRD spectrum of the product obtained in Comparative Example 3;
Figure 12 is the XRD spectrum of the product obtained in Comparative Example 4.

### Detailed description

The specific embodiments of the present application will be described in detail below, but it should be noted that the protection scope of the present application is not limited by these specific embodiments, but is determined by the appended claims.

All publications, patent applications, patents, and other references mentioned in this specification are hereby incorporated by reference. Unless otherwise defined, all technical and scientific terms used in this specification have the meaning commonly understood by those skilled in the art. In case of conflict, the definitions in this specification shall control.

When materials, substances, methods, steps, devices, components, or the like are described herein as being "well-known to those skilled in the art", "prior art", or the like, they are intended to cover those commonly used in the art at the time of filing, and those that are not commonly used at the present time but will become known in the art as being useful for a similar purpose.

In the context of this specification, except for what is explicitly stated, any item or matter not mentioned is directly applicable to those known in the art without any changes. Moreover, any embodiment described herein can be freely combined with one or more other embodiments described herein, and the technical solutions or technical ideas thus formed shall be regarded as the part of the original disclosure or original content of this application and shall not be considered to be new matter that has not been disclosed or expected herein, unless those skilled in the art believe that the combination is obviously unreasonable.

In the context of this specification, unless otherwise stated, the chemical composition of a molecular sieve is calculated in terms of the molar ratio of the corresponding elements as oxides in the highest valence state that can stably exist, i.e. as P₂O₅ for phosphorus (P), as SiO₂ for silicon (Si), as Al₂O₃ for aluminium (Al), as TiO₂ for titanium (Ti), as B₂O₃ for boron (B), as ZrO₂ for zirconium (Zr), as SnO₂ for stannum (Sn), and as Fe₂O₃ for iron (Fe).

In the context of this specification, unless otherwise stated, all percentages, parts, ratios, and the like mentioned in this specification are based on the weight, unless the basis on the weight does not conform to the common understanding of those skilled in the art.

In the context of this specification, the symbol "/" is usually understood as "and/or", for example, the expression "more/greater" means "more and/or greater", unless this understanding does not conform to the conventional understanding of those skilled in the art.

In the context of this specification, as C₁-C₆ linear or branched alkyl, methyl, ethyl, propyl, isopropyl, butyl, isobutyl, tert-butyl, sec-butyl, pentyl and isomers thereof, hexyl and isomers thereof can be enumerated. The C₁-C₆ linear or branched alkyl is preferably C₁-C₄ linear or branched alkyl, more preferably C₁-C₃ linear or branched alkyl.

A first aspect of the present invention provides a SCM-38 molecular sieve, wherein the molecular sieve is a phosphorus-aluminium or silicon-phosphorus-aluminium molecular sieve, the SCM-38 molecular sieve shows the following diffraction peak feature in its XRD spectrum: X-ray diffraction peaks appear at 2θ diffraction angles of 7.20±0.1, 10.81±0.1, 11.60±0.1, 14.32±0.1, 21.39±0.1, 21.83±0.1, 27.31±0.1, 28.72±0.1, wherein the most intense peak appears at 2θ of 7.20±0.1.

In an embodiment of the present invention, the SCM-38 molecular sieve has a schematic chemical composition represented by "P₂O₅·Al₂O₃·SiO₂", wherein based on the molar ratio, Al₂O₃:P₂O₅=0.8-1.5:1, preferably 0.8-1.4, more preferably 0.9-1.3; based on the molar ratio, SiO₂:P₂O₅=0-0.1:1, preferably 0-0.09:1, more preferably 0-0.07:1.

As mentioned above, the SCM-38 molecular sieve of the present invention has a schematic chemical composition represented by "P₂O₅·Al₂O₃·SiO₂", however, it may be free of SiO₂, namely SiO₂:P₂O₅=0, in this case, the molecular sieve of the present invention has a schematic chemical composition represented by "P₂O₅·Al₂O₃".

In an embodiment of the present invention, the molecular sieve (SCM-38 molecular sieve) of the present invention may generally further contain a template agent (quaternary ammonium compounds 1 and 2 of the present invention), water, a fluorine component derived from the fluorine source, and the like, for example, those filled in its channels. However, the present invention does not consider it necessary to limit the amounts of water, template agent, and fluorine component, as the properties of the phosphorus-aluminium molecular sieve or the silicon-phosphor-aluminium molecular sieve of the present invention are mainly determined by the ratio between silicon, phosphorus, and aluminium, and these elements exist in the form of oxides. Therefore, the composition of the molecular sieve of the present invention is only represented as oxides.

In an embodiment of the present invention, the molecular sieve of the present invention has a triangle morphology.

In an embodiment of the present invention, the XRD spectrum of the molecular sieve of the present invention has X-ray diffraction peaks as shown in the following table:

| 2θ(°) | relative intensity, [(I/I₀)×100] |
|---|---|
| 7.20±0.1 | 100 |
| 10.81±0.1 | 5-50 |
| 11.60±0.1 | 5-50 |

| | |
|---|---|
| 14.32±0.1 | 5-50 |
| 21.39±0.1 | 5-50 |
| 21.83±0.1 | 5-50 |
| 27.31±0.1 | 5-50 |
| 28.72±0.1 | 5-50 |

The second aspect of the present invention provides a process for preparing the SCM-38 molecular sieve, comprising the following steps:
a) mixing an aluminophosphate precursor, a quaternary ammonium compound 1, a quaternary ammonium compound 2, a fluorine source and water as well as an optional aluminium source, and an optional silicon source to produce a synthesis mother liquor;
b) crystallizing the synthesis mother liquor obtained in step a) to produce a SCM-38 molecular sieve, wherein, the quaternary ammonium compound 1 is a compound represented by the following formula (I), 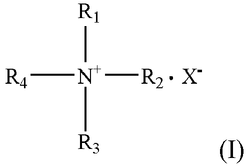
   wherein, R₁, R₂, R₃ and R₄ are each independently selected from C₁-C₆ linear or branched alkyl, X⁻represents a counter ion,
   the quaternary ammonium compound 2 is a compound represented by the following formula (II), 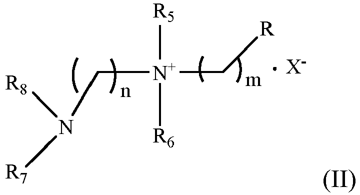
   wherein, R₅, R₆, R₇ and R₈ are each independently selected from C₁-C₆ linear or branched alkyl, R represents hydrogen or hydroxy, X⁻ represents a counter ion, m represents an integral of 1-4, n represents an integral of 5-10,
   the X⁻ in at least one of the compound represented by formula (I) and the compound represented by formula (II) represents hydroxyl group.

In an embodiment of the present invention, preferably R₁, R₂, R₃ and R₄ are each independently selected from C₁-C₄ linear or branched alkyl, more preferably C₁-C₃ linear or branched alkyl. As specific examples of R₁, R₂, R₃ and R₄, methyl, ethyl, propyl, isopropyl, butyl, isobutyl, tert-butyl, sec-butyl, pentyl and isomers thereof, hexyl and isomers thereof can be enumerated.

In an embodiment of the present invention, preferably R₅, R₆, R₇ and R₈ are each independently selected from C₁-C₄ linear or branched alkyl, more preferably C₁-C₃ linear or branched alkyl. As specific examples of R₅, R₆, R₇ and R₈, methyl, ethyl, propyl, isopropyl, butyl, isobutyl, tert-butyl, sec-butyl, pentyl and isomers thereof, hexyl and isomers thereof can be enumerated.

In an embodiment of the present invention, m is preferably an integral of 1-3, more preferably an integral of 1-2.

In an embodiment of the present invention, n is preferably an integral of 5-8, more preferably an integral of 5-6.

In an embodiment of the present invention, the above-mentioned counter ion (X⁻) in the compound represented by formula (I) and the compound represented by formula (II) can be a conventional anion that is compatible with quaternary ammonium ions, including but not limited to halogen (fluorine, chlorine, bromine, iodine) anions, nitrate group, hydroxyl group and the like.

In an embodiment of the present invention, the above-mentioned counter ion (X⁻) in at least one of the compound represented by formula (I) and the compound represented by formula (II) represents hydroxyl group. That is, at least one of the compound represented by formula (I) and the compound represented by formula (II) is a quaternary ammonium hydroxide salt.

In an embodiment of the present invention, the quaternary ammonium compound 1 of the present invention is preferably one or more of tetramethylammonium hydroxide, tetraethylammonium hydroxide, tetrapropylammonium hydroxide and others.

In an embodiment of the present invention, the quaternary ammonium cation in the quaternary ammonium compound 2 of the present invention is preferably 6-N,N-dimethylaminohexyl-2-hydroxyethyl dimethylammonium ion or 6-N,N-dimethylaminohexyl-ethyl dimethyl ammonium ion, the chemical structural formula of which is shown as follows:

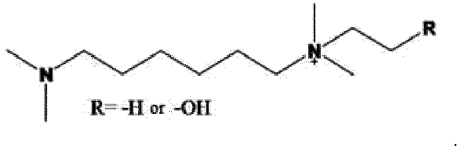

In an embodiment of the present invention, in the synthesis mother liquor of step a), the molar ratio of each material is as follows: silicon source as SiO₂, aluminium source as Al₂O₃, aluminophosphate precursor as Al₂O₃ and P₂O₅, fluorine source as HF, and water as H₂O, (0-0.5)SiO₂:(0.8-5)Al₂O₃:1P₂O₅:(0.01-0.9) quaternary ammonium compound 1:(0.9-5) quaternary ammonium compound 2:(0.1-5)HF:(10-500)H₂O, preferably (0-0.3)SiO₂:(0.85-2)Al₂O₃:1P₂O₅:(0.003-0.85) quaternary ammonium compound 1:(0.95-3) quaternary ammonium compound 2:(0.3-3)HF:(20-300)H₂O, more preferably (0-0.1)SiO₂:(0.0.86-1.5)Al₂O₃:1P₂O₅:(0.05-0.8) quaternary ammonium compound 1:(1-2) quaternary ammonium compound 2:(0.5-1.8)HF:(50-130)H₂O. In the present invention, the amount of the aluminium source is determined based on the amount of Al₂O₃ in the aluminophosphate precursor. That is, when the amount of Al₂O₃ provided by the aluminophosphate precursor meets the above ratio requirements, no aluminium source is added; and when the amount of Al₂O₃ provided by the aluminophosphate precursor cannot meet the above ratio requirements, an aluminium source is added to meet the above ratio requirements.

In an embodiment of the present invention, the aluminium source is one or more of pseudo-boehmite, aluminium isopropoxide, alumina sol, alumina, aluminium chloride, aluminium sulfate, hydrated alumina, sodium metalluminate, aluminium hydroxide, and the like.

In an embodiment of the present invention, the silicon source is one or more of silicasol, fumed silica, ethyl orthosilicate, silicic acid, silica gel and the like.

In an embodiment of the present invention, the fluorine source is one or more of hydrofluoric acid and ammonium fluoride.

In an embodiment of the present invention, there is no special limitation on the order of adding each material in step a). It is preferable to first mix water, the optional aluminum source, the optional silicon source, and the phosphoaluminate precursor evenly, then successively add the quaternary ammonium compound 2 and the quaternary ammonium compound 1 and mix evenly, and then add the fluorine source. Herein, stirring can also be carried out as needed.

In an embodiment of the present invention, in step b), the crystallization conditions are as follows: in a first stage: the crystallization temperature is 120°C-150°C, the crystallization time is 24-84 hours, preferably, the crystallization temperature is 120°C-140°C, the crystallization time is 48-80 hours; in a second stage: the crystallization temperature is 170°C-200°C, the crystallization time is 2-24 hours, preferably, the crystallization temperature is 180°C-200°C, the crystallization time is 4-18 hours.

In an embodiment of the present invention, after step b) i.e., the crystallization step is completed, the SCM-38 molecular sieve product can be separated from the obtained mixture using any commonly known separation method, such as centrifugal separation, washing and drying. Herein, the separation, washing, and drying can be carried out in any conventional known manner in the art, wherein the separation is for example centrifugation, filtering, or filtering by suction. The drying temperature can be 40-120°C, preferably 50-80°C; and the drying time is 8-48 hours, preferably 12-24 hours. The drying can be carried out under atmospheric pressure or under reduced pressure. To save energy, atmospheric pressure is often chosen.

In an embodiment of the present invention, the aluminophosphate precursor has a schematic chemical composition as represented by the formula "Al₂O₃:xP₂O₅", wherein, 0.7≤x≤2.5, preferably 0.8≤x≤2. In an embodiment of the present invention, the XRD spectrum of the aluminophosphate precursor has X-ray diffraction peaks as shown in the following table:

| 2θ(°) | relative intensity, [(I/I₀)×100] |
|---|---|
| 7.59±0.2 | 100 |
| 10.81±0.1 | 5-50 |
| 16.52±0.1 | 5-50 |
| 17.97±0.1 | 5-50 |
| 23.34±0.05 | 5-50 |
| 34.74±0.05 | 5-50 |

In an embodiment of the present invention, the XRD spectrum of the aluminophosphate precursor further has X-ray diffraction peaks as shown in the following table:

| 2θ(°) | relative intensity, [(I/I₀)×100] |
|---|---|
| 14.25±0.1 | 5-50 |
| 21.01±0.1 | 10-20 |
| 24.27±0.05 | 5-50 |
| 26.05±0.05 | 5-50 |
| 27.82±0.05 | 5-50 |
| 28.15±0.02 | 5-50 |
| 30.03±0.02 | 5-50 |

In an embodiment of the present invention, the XRD spectrum of the aluminophosphate precursor further has X-ray diffraction peaks as shown in the following table:

| 2θ(°) | relative intensity, [(I/I₀)×100] |
|---|---|
| 12.09±0.1 | 5-50 |
| 19.77±0.1 | 5-50 |
| 31.33±0.01 | 5-50 |
| 38.29±0.01 | 5-50 |

In an embodiment of the present invention, the process for preparing the aluminophosphate precursor comprises the following steps:
a step of mixing aluminium source, phosphorus source, organic template agent RA and organic template agent RB, solvent S1, solvent S2 and solvent S3 to obtain a mother liquor mixture; and
a step of crystallizing the mother liquor mixture to obtain an aluminophosphate precursor.

In an embodiment of the present invention, in the process for preparing the aluminophosphate precursor, the organic templet agent RA is one or more of quaternary ammonium salt or quaternary ammonium hydroxide; RB is one or more of imidazole or pyrrolidine derivatives.

In an embodiment of the present invention, in the process for preparing the aluminophosphate precursor, the solvent S1 is one or more of amido solvents; the solvent S2 is one or more of cyclic organic solvents; the S3 is one or more of water or C₁-C₆ alkanols.

In an embodiment of the present invention, in the process for preparing the aluminophosphate precursor, the organic templet agent RA is one or more of tetraethylammonium bromide, tetraethylammonium hydroxide, tetrapropylammonium bromide, tetrapropylammonium hydroxide, tetrabutylammonium bromide, and tetrabutylammonium hydroxide; the organic templet agent RB is one or more of imidazole, 2-methylimidazole, 4-methylimidazole, 1-(3-aminopropyl)imidazole, 2-ethyl-4-methylimidazole, pyrrolidine, 1-(3-pyrrolidine)pyrrolidine, and N-ethyl-2-aminomethylpyrrolidine.

In an embodiment of the present invention, in the process for preparing the aluminophosphate precursor, the solvent S1 is one or more of N,N-dimethyl formamide, N,N-dimethyl acetamide, N,N-diethyl formamide and N,N-dibutyl formamide; the solvent S2 is one or more of 1,4-dioxane, cyclohexane, cyclohexanone and; the solvent S3 is one or more of methanol, ethanol, ethylene glycol, butanol, cyclohexanol and water.

In an embodiment of the present invention, in the process for preparing the aluminophosphate precursor, the organic templet agent RA is preferably one or more of tetraethylammonium bromide, tetraethylammonium hydroxide, tetrapropylammonium hydroxide and tetrabutylammonium hydroxide; the organic templet agent RB is preferably one or more of 1-(3-aminopropyl)imidazole, 2-ethyl-4-methylimidazole, and N-ethyl-2-aminomethylpyrrolidine.

In an embodiment of the present invention, in the process for preparing the aluminophosphate precursor, the solvent S1 is preferably one or more of N,N-dimethyl acetamide and N,N-dibutyl formamide; the solvent S2 is preferably one or two of 1,4-dioxane and cyclohexanone; the solvent S3 is preferably one or two of ethanol and water, further preferably deionized water.

In an embodiment of the present invention, in the process for preparing the aluminophosphate precursor, in the preparation step of the mother liquor mixture, the molar composition of aluminium source as Al₂O₃, phosphorus source as P₂O₅, organic templet agent RA+RB, and solvent S1+S2+S3 is as follows: P₂O₅/Al₂O₃=0.75-2.5, preferably 0.8-2.3; templet agent RA+RB/Al₂O₃=1-80, preferably 5-50; solvent S1+S2+S3/Al₂O₃=5-500, preferably 35-120.

In an embodiment of the present invention, in the process for preparing the aluminophosphate precursor, the molar ratio of the organic templet agent RA to the organic templet agent RB is 0.01 - 1:1, preferably 0.1 - 0.5:1.

In an embodiment of the present invention, in the process for preparing the aluminophosphate precursor, the molar ratio of the solvent S1, the solvent S2, and the solvent S3 is 1:0.01-1:1-100, preferably 1:0.05-0.6:10-80.

In an embodiment of the present invention, in the process for preparing the aluminophosphate precursor, the aluminium source is one or more of aluminium isopropoxide, aluminate, metaaluminate, aluminium salt, aluminium hydroxide, aluminium oxide and aluminium-containing mineral, preferably one or two of aluminate and metaaluminate.

In an embodiment of the present invention, in the process for preparing the aluminophosphate precursor, the phosphorus source is at least one of phosphoric acid, ammonium monohydrogen phosphate and ammonium dihydrogen phosphate, preferably orthophosphoric acid.

In an embodiment of the present invention, in the process for preparing the aluminophosphate precursor, the stirring and the settling are performed by the crystallization. The stirring time is 0.5-5 hours, and the settling time is 1-12 hours.

In an embodiment of the present invention, in the process for preparing the aluminophosphate precursor, in the preparation step of the mother liquor mixture, the mixing order of the raw materials is not particularly limited. Preferably, the aluminum source, the phosphorus source, and the organic templet agent are dispersed in a mixture of one or two of solvents S1, S2 and S3 (for example in solvent S3, and then the resulting mixture is stirred and settled, then the other unadded solvent of S1, S2 and S3 (for example a mixture of solvents S1 and S2) is added. The stirring time is 0.5-5 hours, and the settling time is 1-12 hours.

In an embodiment of the present invention, in the process for preparing the aluminophosphate precursor, after obtaining the mother liquor mixture and before the crystallization, the mother liquor mixture can be heat-treated, the heat treatment temperature can be 40-100°C, preferably 50-90°C; the heat treatment time can be 2-14 hours, preferably 4-12 hours. Herein, stirring can also be carried out as needed.

In an embodiment of the present invention, in the process for preparing the aluminophosphate precursor, the conditions for crystallization comprise: the crystallization temperature is 120-200°C, preferably 140-180°C, more preferably 140-160°C; the crystallization time is 1-5 days, preferably 3-5 days, more preferably 4-5 days.

In an embodiment of the present invention, in the process for preparing the aluminophosphate precursor, the crystallization is followed by conventional post-treatment, such as steps of filtering, washing, and drying to prepare the molecular sieve. The filtering, washing, and drying can be carried out in any conventional known manner in the art, wherein the separation is for example centrifugation, filtering, or filtering by suction. The drying temperature can be 40-120°C, preferably 50-80°C; and the drying time is 8-48 hours, preferably 12-24 hours. The drying can be carried out under atmospheric pressure or under reduced pressure. To save energy, atmospheric pressure is often chosen.

In an embodiment of the present invention, the aluminophosphate precursor of the present invention is SCM-34 molecular sieve.

In an embodiment of the present invention, the aluminophosphate precursor of the present invention can be prepared according to the preparation method disclosed in WO2022052967A1.

The third aspect of the present invention provides a molecular sieve composition, comprising the SCM-38 molecular sieve according to any one of the preceding aspects or the SCM-38 molecular sieve prepared with the preparation process according to any one of the preceding aspects, as well as a binder. In an embodiment of the present invention, the binder is not particularly limited and can be a conventional binder in the art, such as one or more of nano-silica, alumina, diatomite, hollow ceramic balls and the like.

The fourth aspect of the present invention provides a catalyst for producing dimethyl ether from methanol, which contains the above-mentioned SCM-38 molecular sieve of the present invention, or the SCM-38 molecular sieve prepared with the preparation process according to any one of the preceding aspects, or the above-mentioned molecular sieve composition of the present invention.

A fifth aspect of the present invention provides a method for producing dimethyl ether from methanol, which method comprises a step of converting methanol to dimethyl ether in the presence of a catalyst, wherein the catalyst contains the above-mentioned SCM-38 molecular sieve of the present invention, or the SCM-38 molecular sieve prepared with the preparation process according to any one of the preceding aspects, or the above-mentioned molecular sieve composition of the present invention. The SCM-38 molecular sieve of the present invention with special morphology is a new molecular sieve, which has a unique XRD graph and special morphology, enriching the types of molecular sieves and the appearance and morphology of molecular sieves.

### Example

The technical solutions of the present application will be further explained in detail through examples, but the protection scope of the present invention is not limited to these examples.

In the following examples and comparative examples, unless otherwise specified, the reagents and raw materials used are commercially available products with analytical purity.

Specific conditions that are not indicated for experimental methods in the following examples and comparative examples should be selected according to conventional manners and conditions, or according to product specifications.

In the present invention, the structure of the molecular sieve is determined by the X-ray diffraction pattern (XRD). The X-ray diffraction pattern (XRD) of the molecular sieve is determined with an X-ray powder diffractometer (XRD), Model X'pert Pro of PAnalytical Corporation of the Netherlands, using Cu-Kα ray source, Kα1 wavelength λ=1.5405980 Angstroms (Å), nickel filter, working voltage 40kV, electric current 40mA, and scanning range 3-50°.

In the present invention, the compositions of SiO₂, Al₂O₃ and P₂O₅ in the molecular sieve are measured using the ICP method. The element ratio in the sample is analyzed using an inductively coupled plasma emission spectrometer, Varian Analytical 725-ES model, from Varian Company of the United States.

### Example 1

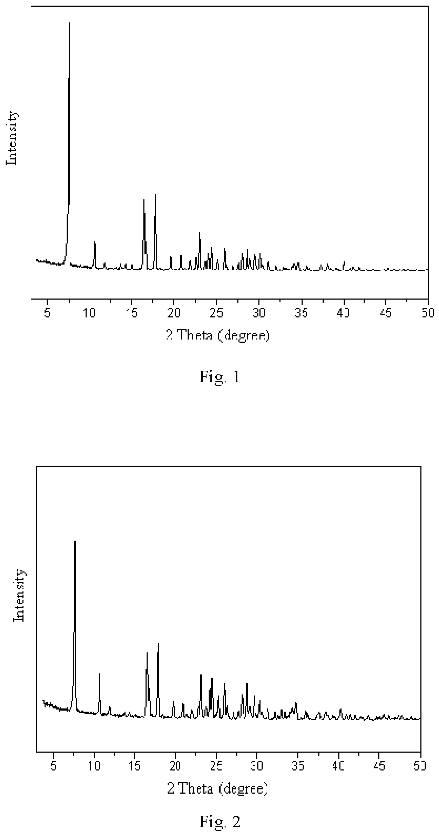

38g of aluminum nitrate [Al(NO3)3·9H₂O] was dissolved in 43mL of deionized water, and 25.2g of phosphoric acid (purity≥85wt%), 151g of tetrabutylammonium hydroxide (40wt% aqueous solution) and 145.6g of 1-(3-aminopropyl)imidazole were added under stirring. The resulting mixture was stirred for 0.5h and settled for 12 hours to obtain solution A. Then 16 mL of N,N-dibutylformamide and 4.6 mL of cyclohexanone were added to solution A, the resulting mixture was stirred for 3.5h and then heat-treated at 90°C for 8 hours to form a uniform mother liquor mixture B, wherein the molar ratio of aluminium source as Al₂O₃, phosphorus source as P₂O₅, total template agent and total solvent was: Al₂O₃:P₂O₅:total template agent:total solvent= 1:2.1:7:40, templet agent RA (tetrabutylammonium hydroxide):templet agent RB (1-(3-aminopropyl)imidazole)= 0.2 (molar ratio), solvent S1 (N,N-dibutyl formamide): solvent S2 (cyclohexanone):solvent S3 (water)= 1:0.5:78.5 (molar ratio). The above mother liquor mixture B was placed in a crystallization kettle lined with tetrafluoroethylene and crystallized at 140°C for 5 days. The product was filtered, washed and dried at 80°C for 24 hours to obtain an aluminophosphate precursor, which was marked as A for use, wherein the aluminophosphate precursor A has a schematic chemical composition as represented by the formula "Al₂O₃:xP₂O₅", x=2. The XRD spectrum of the aluminophosphate precursor A is shown in Figure 1, i.e. it has the X-ray diffraction peaks shown in Table 1 below:

**Table 1**

| 2θ(°) | relative intensity, [(I/I₀)×100] |
|---|---|
| 7.41 | 100 |
| 10.71 | 25 |
| 12.00 | 6 |
| 14.21 | 5 |
| 16.45 | 32 |
| 17.94 | 36 |
| 19.73 | 9 |
| 21.00 | 10 |
| 23.33 | 22 |
| 24.27 | 18 |
| 26.03 | 18 |
| 27.80 | 11 |
| 28.15 | 13 |
| 30.01 | 10 |
| 31.34 | 5 |
| 34.71 | 7 |
| 38.28 | 6 |

### Example 2

33.3g of aluminum sulfate [Al₂(SO₄)₃·18H₂O] was dissolved in 66.3mL of water, and 5.2g of phosphoric acid (purity≥85wt%), 117.0g of tetrabutyl ammonium hydroxide (40wt% aqueous solution) and 102.6g of 1-(3-aminopropyl)imidazole were added under stirring. The resulting mixture was stirring for 3 hours and settled for 6 hours to obtain a solution A. Then 255ml of N,N-dibutyl formamide and 48ml of cyclohexanone were added to solution A, the resulting mixture was stirred for 3.5h and then heat-treated at 80°C for 12 hours to form a uniform mother liquor mixture B, wherein the molar ratio of aluminium source as Al₂O₃, phosphorus source as P₂O₅, total template agent and total solvent was: Al₂O₃:P₂O₅:total template agent:total solvent=1:0.9:10:80, templet agent RA (tetrabutylammonium hydroxide):templet agent RB (1-(3-aminopropyl)imidazole)= 0.22 (molar ratio), solvent S1 (N,N-dibutyl formamide): solvent S2 (cyclohexanone): solvent S3 (water)=1:0.3 :48 (molar ratio). The above mother liquor mixture B was placed in a crystallization kettle lined with tetrafluoroethylene and crystallized at 140°C for 5 days. The product was filtered, washed and dried at 80°C for 24 hours to obtain an aluminophosphate precursor, which was marked as B for use, wherein the aluminophosphate precursor B has a schematic chemical composition as represented by the formula "Al₂O₃:xP₂O₅", x=0.92. The XRD spectrum of the aluminophosphate precursor A is shown in Figure 2, i.e. it has the X-ray diffraction peaks shown in Table 2 below:

**Table 2**

| 2θ(°) | relative intensity, [(I/I₀)×100] |
|---|---|
| 7.49 | 100 |
| 10.77 | 35 |
| 12.01 | 5 |
| 14.18 | 15 |
| 16.43 | 38 |
| 17.87 | 40 |
| 19.70 | 8 |
| 20.96 | 12 |
| 23.32 | 30 |
| 24.22 | 26 |
| 26.03 | 23 |
| 27.80 | 17 |
| 28.15 | 20 |
| 30.01 | 18 |
| 31.32 | 8 |
| 34.74 | 8 |
| 38.28 | 5 |

### Example 3

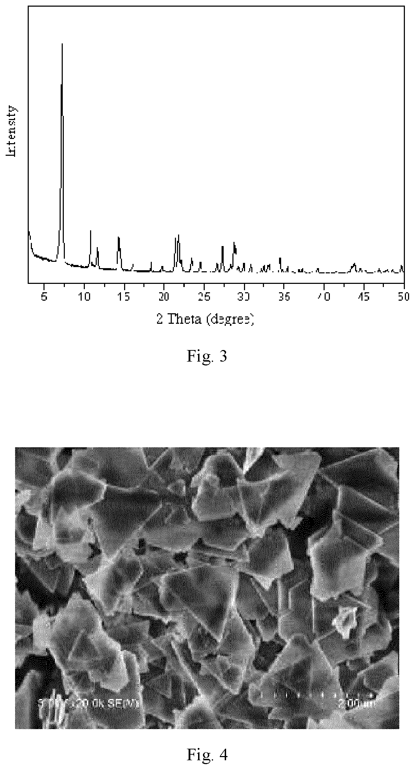

A measured amount of deionized water was taken; 3.1g of aluminium isopropoxide and 6g of aluminophosphate precursor A were added, and the resulting mixture was stirred at room temperature for 2 hours; then 4.75g of 6-N,N-dimethylaminohexyl-2-hydroxyethyldimethylammonium bromide (quaternary ammonium compound 2) was added, then 4.4g of 25wt% tetraethylammonium hydroxide (quaternary ammonium compound 1) solution was added, the resulting mixture was stirred at room temperature for 3 hours and then 1g of 40wt% HF solution was added, finally the molar ratio of components in the mixed solution was: 1 Al₂O₃:1 P₂O₅:0.5 quaternary ammonium compound 1:1 quaternary ammonium compound 2:1.3 HF:88 H₂O. The resulting mixture was stirred evenly; then placed in an autoclave lined with tetrafluoroethylene; crystallized at 140°C for 60 hours, then heated up to 180°C, and crystallized at 180°C for 8 hours; cooled, centrifuged and washed (the centrifuging and washing operation was repeated 2-3 times); and dried at 80°C for 24 hours to obtain SCM-38 molecular sieve, which has a schematic chemical composition represented by "P₂O₅·Al₂O₃", based on the molar ratio of Al₂O₃:P₂O₅=1.22:1. The SEM of the obtained SCM-38 molecular sieve is shown in Figure 4. Its morphology is a trigon morphology. Its XRD spectrum is shown in Figure 3, and has the main X-ray diffraction peaks shown in Table 3 below:

**Table 3**

| 2θ(°) | relative intensity, [(I/I₀)×100] |
|---|---|
| 7.19 | 100 |
| 10.82 | 17 |
| 11.60 | 9 |
| 14.32 | 15 |
| 21.39 | 15 |
| 21.80 | 17 |
| 27.30 | 12 |
| 28.72 | 13 |

### Example 4

A measured amount of deionized water was taken; 1.24g of pseudo-boehmite and 6g of aluminophosphate precursor A were added, and the resulting mixture was stirred at room temperature for 3 hours; then 4.75g of 6-N,N-dimethylaminohexyl-2-hydroxyethyldimethylammonium bromide (quaternary ammonium compound 2) was added, then 4.4g of 25wt% tetraethylammonium hydroxide (quaternary ammonium compound 1) solution was added, the resulting mixture was stirred at room temperature for 3 hours and then 1g of 40wt% HF solution was added, finally the molar ratio of components in the mixed solution was: 1 Al₂O₃:1 P₂O₅:0.5 quaternary ammonium compound 1:1 quaternary ammonium compound 2:1.3 HF:88 H₂O. The resulting mixture was stirred evenly; then placed in an autoclave lined with tetrafluoroethylene; crystallized at 140°C for 66 hours, then heated up to 180°C, and crystallized at 180°C for 8 hours; cooled, centrifuged and washed (the centrifuging and washing operation was repeated 2-3 times); and dried at 80°C for 24 hours to obtain SCM-38 molecular sieve, which has a schematic chemical composition represented by "P₂O₅·Al₂O₃", based on the molar ratio of Al₂O₃:P₂O₅=0.99:1. The SEM image of the molecular sieve is similar to that shown in Figure 4. Its morphology is a trigon morphology, and the XRD spectrum of the obtained SCM-38 molecular sieve has the main X-ray diffraction peaks shown in Table 4 below:

**Table 4**

| 2θ(°) | relative intensity, [(I/I₀)×100] |
|---|---|
| 7.20 | 100 |
| 10.83 | 18 |
| 11.63 | 7 |
| 14.33 | 18 |
| 21.37 | 13 |
| 21.83 | 24 |
| 27.31 | 10 |
| 28.69 | 26 |

### Example 5

A measured amount of deionized water was taken; 3.7g of aluminium isopropoxide and 6g of aluminophosphate precursor A were added, and the resulting mixture was stirred at room temperature for 3 hours; then 6.9g of 6-N,N-dimethylaminohexyl-2-hydroxyethyldimethylammonium bromide (quaternary ammonium compound 2) was added, then 4.4g of 25wt% tetraethylammonium hydroxide (quaternary ammonium compound 1) solution was added, the resulting mixture was stirred at room temperature for 3 hours and then 1g of 40wt% HF solution was added, finally the molar ratio of components in the mixed solution was: 1.1 Al₂O₃:1 P₂O₅:0.5 quaternary ammonium compound 1:1 quaternary ammonium compound 2:1.3 HF:88 H₂O. The resulting mixture was stirred evenly; then placed in an autoclave lined with tetrafluoroethylene; crystallized at 140°C for 48 hours, then heated up to 180°C, and crystallized at 180°C for 8 hours; cooled, centrifuged and washed (the centrifuging and washing operation was repeated 2-3 times); and dried at 80°C for 24 hours to obtain SCM-38 molecular sieve, which has a schematic chemical composition represented by "P₂O₅·Al₂O₃", based on the molar ratio of Al₂O₃:P₂O₅=1.15:1. The SEM image of the molecular sieve is similar to that shown in Figure 4. Its morphology is a trigon morphology, and the XRD spectrum of the obtained SCM-38 molecular sieve has the main X-ray diffraction peaks shown in Table 5 below:

**Table 5**

| 2θ(°) | relative intensity, [(I/I₀)×100] |
|---|---|
| 7.21 | 100 |
| 10.83 | 18 |
| 11.63 | 8 |
| 14.31 | 21 |
| 21.40 | 15 |
| 21.84 | 25 |
| 27.32 | 13 |
| 28.73 | 27 |

### Example 6

A measured amount of deionized water was taken; 4.3g of aluminium isopropoxide and 6g of aluminophosphate precursor A were added, and the resulting mixture was stirred at room temperature for 2 hours; then 4.75g of 6-N,N-dimethylaminohexyl-2-hydroxyethyldimethylammonium bromide (quaternary ammonium compound 2) was added, then 6.6g of 25wt% tetraethylammonium hydroxide (quaternary ammonium compound 1) solution was added, the resulting mixture was stirred at room temperature for 3 hours and then 1g of 40wt% HF solution was added, the molar ratio of components was: 1.2 Al₂O₃:1 P₂O₅:0.7 quaternary ammonium compound 1:1 quaternary ammonium compound 2:1.3 HF:100 H₂O. The resulting mixture was stirred evenly; then placed in an autoclave lined with tetrafluoroethylene; crystallized at 140°C for 66 hours; then heated up to 180°C and crystallized at 180°C for 12 hours; cooled, centrifuged and washed (the centrifuging and washing operation was repeated 2-3 times), dried at 80°C for 24 hours to obtain SCM-38 molecular sieve, which has a schematic chemical composition represented by "P₂O₅·Al₂O₃", based on the molar ratio of Al₂O₃:P₂O₅= 1.14:1. The SEM image of the molecular sieve is similar to that shown in Figure 4. Its morphology is a trigon morphology, and the XRD spectrum of the obtained SCM-38 molecular sieve has the main X-ray diffraction peaks shown in Table 6 below:

**Table 6**

| 2θ(°) | relative intensity, [(I/I₀)×100] |
|---|---|
| 7.19 | 100 |
| 10.79 | 16 |
| 11.59 | 8 |
| 14.30 | 20 |
| 21.30 | 12 |
| 21.81 | 22 |
| 27.29 | 9 |
| 28.69 | 23 |

### Example 7

A measured amount of deionized water was taken; 4.65g of aluminium isopropoxide and 6g of aluminophosphate precursor A were added, the resulting mixture was stirred at room temperature for 2 hours; then 8.2g of 6-N,N-dimethylaminohexyl-2-hydroxyethyldimethylammonium bromide (quaternary ammonium compound 2) was added, then 6.6g of 25wt% tetraethylammonium hydroxide (quaternary ammonium compound 1) solution was added, the resulting mixture was stirred at room temperature for 3 hours and then 1.3g of 40wt% HF solution was added, the molar ratio of components was: 1.3 Al₂O₃:1 P₂O₅:0.7 quaternary ammonium compound 1:1.8 quaternary ammonium compound 2:1.7 HF:110 H₂O. The resulting mixture was stirred evenly; then placed in an autoclave lined with tetrafluoroethylene; crystallized at 140°C for 66 hours; then heated up to 180°C and crystallized at 180°C for 4 hours; cooled, centrifuged and washed (the centrifuging and washing operation was repeated 2-3 times), dried at 80°C for 24 hours to obtain SCM-38 molecular sieve, which has a schematic chemical composition represented by "P₂O₅·Al₂O₃", based on the molar ratio of Al₂O₃:P₂O₅= 1.09:1. The SEM image of the molecular sieve is similar to that shown in Figure 4. Its morphology is a trigon morphology, and the XRD spectrum of the obtained SCM-38 molecular sieve has the main X-ray diffraction peaks shown in Table 7 below:

**Table 7**

| 2θ(°) | relative intensity, [(I/I₀)×100] |
|---|---|
| 7.20 | 100 |
| 10.82 | 22 |
| 11.60 | 11 |
| 14.33 | 22 |
| 21.38 | 18 |
| 21.82 | 23 |
| 27.27 | 8 |
| 28.70 | 21 |

### Example 8

A measured amount of deionized water was taken; 3.1g of aluminium isopropoxide and 6g of aluminophosphate precursor A were added, the resulting mixture was stirred at room temperature for 2 hours; then 4.75g of 6-N,N-dimethylaminohexyl-2-hydroxyethyldimethylammonium bromide (quaternary ammonium compound 2) was added, then 2.73g of 25wt% tetramethylammonium hydroxide (quaternary ammonium compound 1) solution was added, the resulting mixture was stirred at room temperature for 3 hours and then 1g of 40wt% HF solution was added, the molar ratio of components was: 1 Al₂O₃:1 P₂O₅:0.5 quaternary ammonium compound 1:1 quaternary ammonium compound 2:1.3 HF:88 H₂O. The resulting mixture was stirred evenly; then placed in an autoclave lined with tetrafluoroethylene; crystallized at 140°C for 66 hours; then heated up to 180°C and crystallized at 180°C for 8 hours; cooled, centrifuged and washed (the centrifuging and washing operation was repeated 2-3 times), dried at 80°C for 24 hours to obtain SCM-38 molecular sieve, which has a schematic chemical composition represented by "P₂O₅·Al₂O₃", based on the molar ratio of Al₂O₃:P₂O₅=1.06:1. The SEM image of the molecular sieve is similar to that shown in Figure 4. Its morphology is a trigon morphology, and the XRD spectrum of the obtained SCM-38 molecular sieve has the main X-ray diffraction peaks shown in Table 8 below:

**Table 8**

| 2θ(°) | relative intensity, [(I/I₀)×100] |
|---|---|
| 7.23 | 100 |
| 10.82 | 23 |
| 11.63 | 13 |
| 14.34 | 24 |
| 21.39 | 16 |
| 21.81 | 25 |
| 27.33 | 12 |
| 28.71 | 27 |

### Example 9

A measured amount of deionized water was taken; 3.1g of aluminium isopropoxide and 6g of aluminophosphate precursor A were added, and the resulting mixture was stirred at room temperature for 3 hours; then 4.75g of 6-N,N-dimethylaminohexyl-2-hydroxyethyldimethylammonium bromide (quaternary ammonium compound 2) was added, then 6.1g of 25wt% tetrapropylammonium hydroxide (quaternary ammonium compound 1) solution was added, the resulting mixture was stirred at room temperature for 3 hours and then 1g of 40wt% HF solution was added, the molar ratio of components was: 1 Al₂O₃:1 P₂O₅:0.5 quaternary ammonium compound 1:1 quaternary ammonium compound 2:1.3 HF:88 H₂O. The resulting mixture was stirred evenly; then placed in an autoclave lined with tetrafluoroethylene; crystallized at 140°C for 66 hours; then heated up to 180°C and crystallized at 180°C for 8 hours; cooled, centrifuged and washed (the centrifuging and washing operation was repeated 2-3 times), dried at 80°C for 24 hours to obtain SCM-38 molecular sieve, which has a schematic chemical composition represented by "P₂O₅·Al₂O₃", based on the molar ratio of Al₂O₃:P₂O₅= 1.14:1. The SEM image of the molecular sieve is similar to that shown in Figure 4. Its morphology is a trigon morphology, and the XRD spectrum of the obtained SCM-38 molecular sieve has the main X-ray diffraction peaks shown in Table 9 below:

**Table 9**

| 2θ(°) | relative intensity, [(I/I₀)×100] |
|---|---|
| 7.20 | 100 |
| 10.79 | 19 |
| 11.61 | 11 |
| 14.31 | 24 |
| 21.38 | 15 |
| 21.82 | 26 |
| 27.32 | 12 |
| 28.70 | 28 |

### Example 10

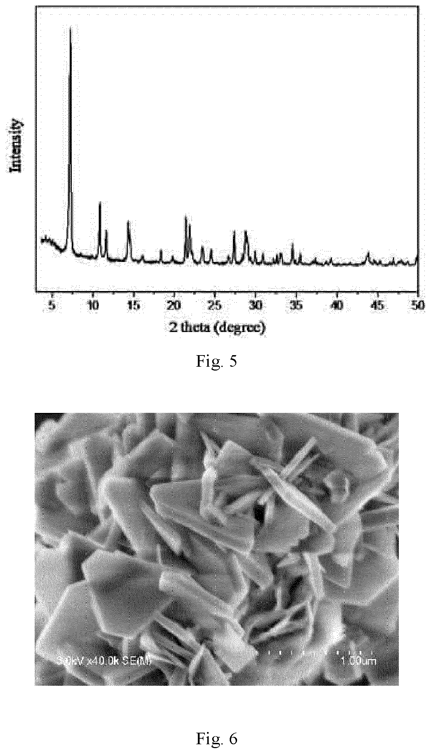

A measured amount of deionized water was taken; 3.1g of aluminium isopropoxide and 6g of aluminophosphate precursor A were added, the resulting mixture was stirred at room temperature for 2 hours; then 1.8g of 4wt% silica sol was added, and the resulting mixture was stirred evenly and then 4.75g of 6-N,N-dimethylaminohexyl-2-hydroxyethyldimethylammonium bromide (quaternary ammonium compound 2) was added, then 4.4g of 25wt% tetraethylammonium hydroxide (quaternary ammonium compound 1) aqueous solution was added, finally the molar ratio of each component in the mixed solution was: 0.08 SiO₂:1 Al₂O₃ :1 P₂O₅:0.5 quaternary ammonium compound 1:1 quaternary ammonium compound 2:1.3 HF:88 H₂O. The resulting mixture was stirred at room temperature for 4 hours and then 1g of 40wt% HF solution was added. The resulting mixture was stirred evenly; then placed in an autoclave lined with tetrafluoroethylene; crystallized at 140°C for 66 hours; then heated up to 180°C and crystallized at 180°C for 8 hours; cooled, centrifuged and washed (the centrifuging and washing operation was repeated 2-3 times), dried at 80°C for 24 hours to obtain SCM-38 molecular sieve, which has a schematic chemical composition represented by "P₂O₅·Al₂O₃·SiO₂", based on the molar ratio of SiO₂:Al₂O₃:P₂O₅=0.07:1.13:1. The SEM image of the molecular sieve is similar to that shown in Figure 6. Its morphology is a trigon morphology. The XRD spectrum of the SCM-38 molecular sieve is shown in Figure 5, and it has the main X-ray diffraction peaks shown in Table 10 below:

**Table 10**

| | |
|---|---|
| 2θ(°) | relative intensity, [(I/I₀)×100] |

| | |
|---|---|
| 7.23 | 100 |
| 10.82 | 25 |
| 11.63 | 15 |
| 14.35 | 18 |
| 21.41 | 21 |
| 21.85 | 17 |
| 27.33 | 15 |
| 28.75 | 15 |

### Example 11

A measured amount of deionized water was taken; 2.48g of aluminium isopropoxide was added, then 6g of aluminophosphate precursor A was added, and the resulting mixture was stirred at room temperature for 3 hours; then 4.75g of 6-N,N-dimethylaminohexyl-2-hydroxyethyldimethylammonium bromide (quaternary ammonium compound 2) was added, then 3.52g of 25wt% tetraethylammonium hydroxide (quaternary ammonium compound 1) aqueous solution was added, the resulting mixture was stirred at room temperature for 3 hours and then 0.43g of 40wt% HF solution was added, finally the molar ratio of each component in the mixed solution was: 0.87 Al₂O₃:1 P₂O₅:0.4 quaternary ammonium compound 1:1 quaternary ammonium compound 2:0.6 HF:55 H₂O. The resulting mixture was stirred evenly; then placed in an autoclave lined with tetrafluoroethylene; crystallized at 130°C for 72 hours, then heated up to 180°C and crystallized at 180°C for 16 hours; cooled, centrifuged and washed (the centrifuging and washing operation was repeated 2-3 times), dried at 80°C for 24 hours to obtain SCM-38 molecular sieve, which has a schematic chemical composition represented by "P₂O₅·Al₂O₃", based on the molar ratio of Al₂O₃:P₂O₅=0.9:1. The SEM image of the molecular sieve is similar to that shown in Figure 4. Its morphology is a trigon morphology, and the XRD spectrum of the obtained SCM-38 molecular sieve has the main X-ray diffraction peaks shown in Table 11 below:

**Table 11**

| 2θ(°) | relative intensity, [(I/I₀)×100] |
|---|---|
| 7.22 | 100 |
| 10.81 | 19 |
| 11.63 | 10 |
| 14.35 | 24 |
| 21.38 | 17 |
| 21.86 | 28 |
| 27.34 | 13 |
| 28.74 | 28 |

### Example 12

A measured amount of deionized water was taken; 3.1g of aluminium isopropoxide was added, then 6g of aluminophosphate precursor A was added, the resulting mixture was stirred at room temperature for 3 hours; then 4.75g of 6-N,N-dimethylaminohexyl-2-hydroxyethyldimethylammonium bromide (quaternary ammonium compound 2) was added, then 4.4g of 25wt% tetraethylammonium hydroxide (quaternary ammonium compound 1) aqueous solution was added, the resulting mixture was stirred at room temperature for 3 hours and then 1g of 40wt% HF solution was added, finally the molar ratio of each component in the mixed solution was: 1 Al₂O₃:1 P₂O₅:0.5 quaternary ammonium compound 1:1 quaternary ammonium compound 2:1.3 HF:120 H₂O. The resulting mixture was stirred evenly; then placed in an autoclave lined with tetrafluoroethylene; crystallized at 120°C for 80 hours, then heated up to 180°C and crystallized at 200°C for 4 hours; cooled, centrifuged and washed (the centrifuging and washing operation was repeated 2-3 times), dried at 80°C for 24 hours to obtain SCM-38 molecular sieve, which has a schematic chemical composition represented by "P₂O₅·Al₂O₃", based on the molar ratio of Al₂O₃:P₂O₅= 1.3:1. The SEM image of the molecular sieve is similar to that shown in Figure 4. Its morphology is a trigon morphology, and the XRD spectrum of the obtained SCM-38 molecular sieve has the main X-ray diffraction peaks shown in Table 12 below:

**Table 12**

| 2θ(°) | relative intensity, [(I/I₀)×100] |
|---|---|
| 7.20 | 100 |
| 10.81 | 16 |
| 11.62 | 7 |
| 14.30 | 20 |
| 21.36 | 13 |
| 21.81 | 24 |
| 27.30 | 11 |
| 28.69 | 26 |

### Example 13

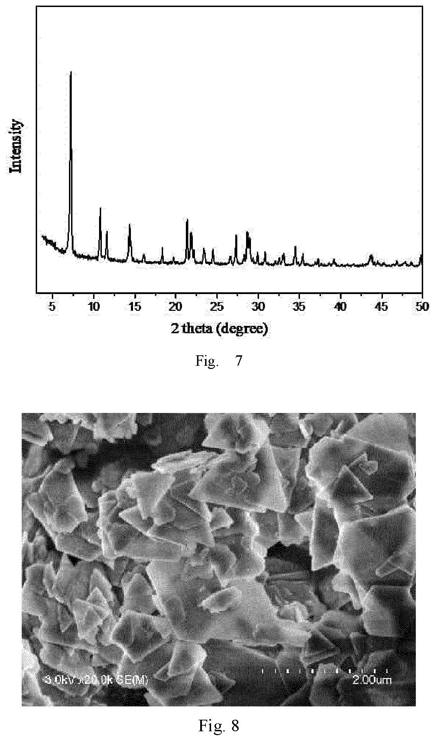

A measured amount of deionized water was taken; 2.73g of aluminophosphate precursor B was added, the resulting mixture was stirred at room temperature for 3 hours; then 4.75g of 6-N,N-dimethylaminohexyl-2-hydroxyethyldimethylammonium bromide (quaternary ammonium compound 2) was added, then 0.5g of 25wt% tetraethylammonium hydroxide (quaternary ammonium compound 1) aqueous solution was added, the resulting mixture was stirred at room temperature for 3 hours and then 1g of 40wt% HF solution was added, finally the molar ratio of each component in the mixed solution was: 1 Al₂O₃:1 P₂O₅:0.06 quaternary ammonium compound 1:1 quaternary ammonium compound 2:1.3 HF:60 H₂O. The resulting mixture was stirred evenly; then placed in an autoclave lined with tetrafluoroethylene; crystallized at 140°C for 66 hours, then heated up to 180°C and crystallized at 180°C for 8 hours; cooled, centrifuged and washed (the centrifuging and washing operation was repeated 2-3 times), dried at 80°C for 24 hours to obtain SCM-38 molecular sieve, which has a schematic chemical composition represented by "P₂O₅·Al₂O₃", based on the molar ratio of Al₂O₃:P₂O₅= 1.12:1. The SEM image of the molecular sieve is similar to that shown in Figure 8. Its morphology is a trigon morphology. The XRD spectrum of the SCM-38 molecular sieve is shown in Figure 7, and it has the main X-ray diffraction peaks shown in Table 13 below:

**Table 13**

| 2θ(°) | relative intensity, [(I/I₀)×100] |
|---|---|
| 7.19 | 100 |
| 10.82 | 29 |
| 11.61 | 17 |
| 14.32 | 21 |
| 21.38 | 24 |
| 21.82 | 17 |
| 27.33 | 17 |
| 28.67 | 19 |

### Example 14

A measured amount of deionized water was taken; 3.1g of aluminium isopropoxide and 6g of aluminophosphate precursor A were added, and the resulting mixture was stirred at room temperature for 2 hours; then 4.72g of 6-N,N-dimethylaminohexyl-propyldimethylammonium bromide (quaternary ammonium compound 2) was added, then 4.4g of 25wt% tetraethylammonium hydroxide (quaternary ammonium compound 1) solution was added, the resulting mixture was stirred at room temperature for 3 hours and then 1g of 40wt% HF solution was added, the molar ratio of components was: 1 Al₂O₃:1 P₂O₅:0.06 R₁:1 R₂:1.3 HF:60 H₂O. The resulting mixture was stirred evenly; then placed in an autoclave lined with tetrafluoroethylene; crystallized at 140°C for 60 hours; then heated up to 180°C and crystallized at 180°C for 8 hours; cooled, centrifuged and washed (the centrifuging and washing operation was repeated 2-3 times), dried at 80°C for 24 hours to obtain SCM-38 molecular sieve, which has a schematic chemical composition represented by "P₂O₅·Al₂O₃", based on the molar ratio of Al₂O₃:P₂O₅= 1.19:1. The SEM of the molecular sieve is similar to that shown in Figure 4. Its morphology is a trigon morphology, and the XRD spectrum of the obtained SCM-38 molecular sieve has the main X-ray diffraction peaks shown in Table 14 below:

**Table 14**

| 2θ(°) | relative intensity, [(I/I₀)×100] |
|---|---|
| 7.19 | 100 |
| 10.81 | 17 |
| 11.58 | 8 |
| 14.30 | 21 |
| 21.38 | 13 |
| 21.81 | 23 |
| 27.30 | 10 |
| 28.71 | 26 |

### Comparative Example 1

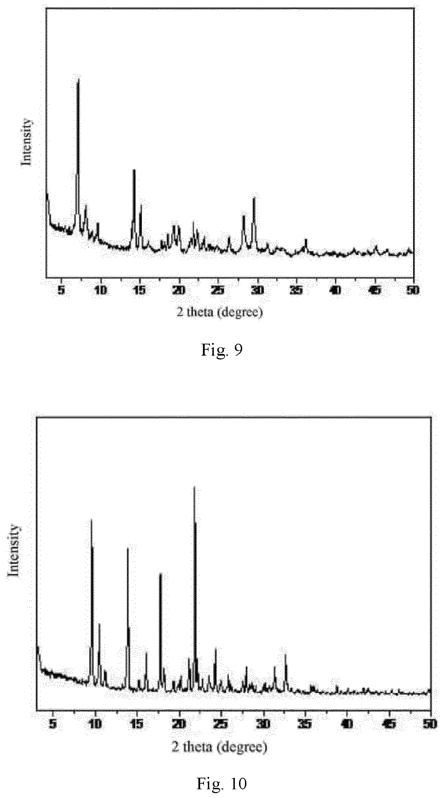

A measured amount of deionized water was taken; 6.2g of aluminium isopropoxide was added, the resulting mixture was stirred at room temperature for 1 hour, then 3.51g of phosphoric acid was added, the resulting mixture was stirred at room temperature for 3 hours; 4.75g of 6-N,N-dimethylaminohexyl-2-hydroxyethyldimethylammonium bromide (quaternary ammonium compound 2) was added, then 4.4g of 25wt% tetraethylammonium hydroxide (quaternary ammonium compound 1) aqueous solution was added, the resulting mixture was stirred at room temperature for 3 hours and then 1g of 40wt% HF solution was added, finally the molar ratio of each component in the mixed solution was: 1 Al₂O₃ :1 P₂O₅:0.5 quaternary ammonium compound 1:1 quaternary ammonium compound 2 :1.3 HF:88 H₂O. The resulting mixture was stirred evenly; then placed in an autoclave lined with tetrafluoroethylene; crystallized at 140°C for 66 hours; then heated up to 180°C and crystallized at 180°C for 8 hours; cooled, centrifuged and washed (the centrifuging and washing operation was repeated 2-3 times), dried at 80°C for 24 hours to obtain a product with the molar ratio of Al₂O₃:P₂O₅=0.97:1. Its XRD graph is shown in Figure 9, demonstrating it is not SCM-38 molecular sieve.

### Comparative Example 2

A measured amount of deionized water was taken; a mixture A of 3.1g of aluminium isopropoxide and 6g of aluminophosphate precursor was added, the resulting mixture was stirred at room temperature for 3 hours; 4.75g of 6-N,N-dimethylaminohexyl-2-hydroxyethyldimethylammonium bromide (quaternary ammonium compound 2) was added, the resulting mixture was stirred at room temperature for 3 hours and then 1g of 40wt% HF solution was added, finally the molar ratio of each component in the mixed solution was: 1 Al₂O₃:1 P₂O₅:0 quaternary ammonium compound 1:1 quaternary ammonium compound 2:1.3 HF:88 H₂O. The resulting mixture was stirred evenly; then placed in an autoclave lined with tetrafluoroethylene; crystallized at 140°C for 66 hours; then heated up to 180°C and crystallized at 180°C for 8 hours; cooled, centrifuged and washed (the centrifuging and washing operation was repeated 2-3 times), dried at 80°C for 24 hours obtain a product with the molar ratio of Al₂O₃:P₂O₅=1:1. Its XRD graph is shown in Figure 10, demonstrating it is not SCM-38 molecular sieve.

### Comparative Example 3

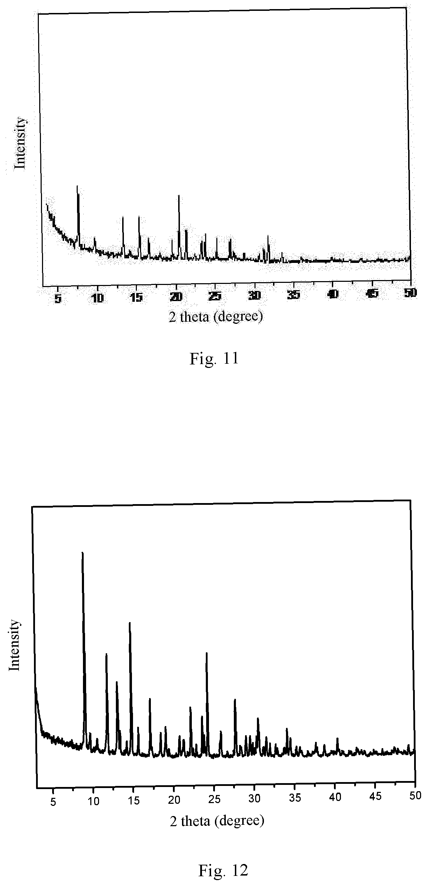

A measured amount of deionized water was taken; a mixture A of 3.1g of aluminium isopropoxide and 6g of aluminophosphate precursor was added, the resulting mixture was stirred at room temperature for 3 hours; 4.75g of 6-N,N-dimethylaminohexyl-2-hydroxyethyldimethylammonium bromide (quaternary ammonium compound 2) was added, then 4.4g of 25wt% tetraethylammonium hydroxide (quaternary ammonium compound 1) aqueous solution was added, finally the molar ratio of components in the mixture system was: 1 Al₂O₃:1 P₂O₅:0.5 quaternary ammonium compound 1:1 quaternary ammonium compound 2:0 HF:88 H₂O. The resulting mixture was stirred at room temperature for 3 hours; then placed in an autoclave lined with tetrafluoroethylene; crystallized at 140°C for 66 hours; then heated up to 180°C and crystallized at 180°C for 8 hours; cooled, centrifuged and washed (the centrifuging and washing operation was repeated 2-3 times), dried at 80°C for 24 hours obtain a product with the molar ratio of Al₂O₃:P₂O₅=1:1. Its XRD graph is shown in Figure 11, demonstrating it is not SCM-38 molecular sieve.

### Comparative Example 4

A measured amount of deionized water was taken; 1.5g of aluminium isopropoxide and 6g of aluminophosphate precursor A were added, and the resulting mixture was stirred at room temperature for 2 hours; then 4.72g of 6-N,N-dimethylaminohexyl-propyldimethylammonium bromide (quaternary ammonium compound 2) was added, then 4.4g of 25wt% tetraethylammonium hydroxide (quaternary ammonium compound 1) solution was added, the resulting mixture was stirred at room temperature for 3 hours and then 1g of 40wt% HF solution was added, finally the molar ratio of components in the mixing system was: 0.7 Al₂O₃:1 P₂O₅:0.5 quaternary ammonium compound 1:1 quaternary ammonium compound 2:1.3 HF: 88H₂O. The resulting mixture was stirred evenly; then placed in an autoclave lined with tetrafluoroethylene; crystallized at 140°C for 60 hours; then heated up to 180°C and crystallized at 180°C for 8 hours; cooled, centrifuged and washed (the centrifuging and washing operation was repeated 2-3 times), dried at 80°C for 24 hours obtain a product with the molar ratio of Al₂O₃:P₂O₅=1:1. Its XRD graph is shown in Figure 12, demonstrating it is not SCM-38 molecular sieve.

### Example 15: Catalytic Activity Evaluation

### Preparation of catalyst 1:

2.5g of SCM-38 prepared in Example 3 was weighed, 1g of alumina was added, and then 5% nitric acid was added. The resulting mixture was thoroughly ground to uniformity, dried at 120°C overnight, then calcined at 460°C for 4 hours under N₂ atmosphere, and then for 4 hours in air atmosphere. The calcined mixture was cooled, tabletted and crushed, and screened. 1g of particles with 20-40 mesh was weighed and used as catalyst 1.

### Preparation of catalyst 2:

Catalyst 2 was prepared according to the same manner as the preparation of the above-mentioned catalyst 1 except that SCM-38 molecular sieve prepared in Example 10 was used.

### Preparation of catalyst 3:

Catalyst 3 was prepared according to the same manner as the preparation of the above-mentioned catalyst 1 except that the product prepared in Comparative Example 1 was used.

### Catalytic activity evaluation of catalyst 1

Pure methanol was used as raw material, a fixed bed reactor with a diameter of 15mm was used. The catalyst was loaded into the reactor with a small amount of quartz wool was placed above and below the catalyst. The conversion reaction of methanol to dimethyl ether was performed under the conditions of 460°C, WHSV of 1h-1, and nitrogen flow rate of 20 mL/min under atmospheric pressure. The composition of the obtained product was determined, and the results showed that the peak selectivity of dimethyl ether was 94.87%.

### Catalytic activity evaluation of catalyst 2

Catalyst 2 was used to evaluate the activity of catalyst 2 in the same way as that of Catalyst 1. The results showed that the peak selectivity of dimethyl ether was 95.93%.

### Catalytic activity evaluation of catalyst 3

Catalyst 3 was used to evaluate the activity of catalyst 2 in the same way as that of Catalyst 1. The results showed that the peak selectivity of dimethyl ether was 63.42%.

### Industrial applicability

The present invention provides a new phosphorus-aluminium molecular sieve or silicon-phosphor-aluminium molecular sieve. The phosphorus-aluminium molecular sieve or silicon-phosphor-aluminium molecular sieve of the present invention exhibits excellent activity as a catalyst for converting methanol into dimethyl ether.

## Claims

1. A SCM-38 molecular sieve, wherein the molecular sieve is a phosphorus-aluminium or silicon-phosphorus-aluminium molecular sieve, the SCM-38 molecular sieve shows the following diffraction peak feature in its XRD spectrum: X-ray diffraction peaks appear at 2θ diffraction angles of 7.20±0.1, 10.81±0.1, 11.60±0.1, 14.32±0.1, 21.39±0.1, 21.83±0.1, 27.31±0.1, 28.72±0.1, wherein the most intense peak appears at 2θ of 7.20±0.1,
the SCM-38 molecular sieve has a schematic chemical composition represented by "P₂O₅ Al₂O₃·SiO₂", wherein based on the molar ratio, Al₂O₃:P₂O₅=0.8-1.5:1, preferably 0.8-1.4, more preferably 0.9-1.3; SiOz:PzOs=0-0.1:1, preferably 0-0.09:1, more preferably 0-0.07:1.

2. The SCM-38 molecular sieve according to claim 1, **characterized in that** the XRD spectrum of the SCM-38 molecular sieve has X-ray diffraction peaks as shown in the following table:
| 2θ(°) | relative intensity, [(I/I₀)×100] |
|---|---|
| 7.20±0.1 | 100 |
| 10.81±0.1 | 5-50 |
| 11.60±0.1 | 5-50 |
| 14.32±0.1 | 5-50 |
| 21.39±0.1 | 5-50 |
| 21.83±0.1 | 5-50 |
| 27.31±0.1 | 5-50 |
| 28.72±0.1 | 5-50. |

3. The SCM-38 molecular sieve according to claim 1, **characterized in that**, the SCM-38 molecular sieve has a triangle morphology.

4. A process for preparing the SCM-38 molecular sieve according to any of claims 1-3, comprising:
a) mixing an aluminophosphate precursor, a quaternary ammonium compound 1, a quaternary ammonium compound 2, a fluorine source and water as well as an optional aluminium source, and an optional silicon source to produce a synthesis mother liquor;
b) crystallizing the synthesis mother liquor obtained in step a) to produce a SCM-38 molecular sieve, wherein, the quaternary ammonium compound 1 is a compound represented by the following formula (I), 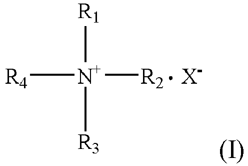
wherein, Ri, R₂, R₃ and R₄ are each independently selected from C₁-C₆ linear or branched alkyl, X⁻represents a counter ion,
the quaternary ammonium compound 2 is a compound represented by the following formula (II), 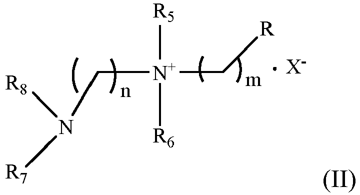
wherein, R₅, R₆, R₇ and R₈ are each independently selected from C₁-C₆ linear or branched alkyl, R represents hydrogen or hydroxy, X⁻ represents a counter ion, m represents an integral of 1-4, n represents an integral of 5-10;
the counter ion X⁻ in at least one of the compound represented by formula (I) and the compound represented by formula (II) represents hydroxyl group,
the aluminophosphate precursor has a schematic chemical composition as represented by the formula "Al₂O₃:xP₂O₅", wherein, 0.7≤x≤2.5, preferably 0.8≤x≤2.

5. The process for preparing the SCM-38 molecular sieve according to claim 4, wherein, the XRD spectrum of the aluminophosphate precursor has X-ray diffraction peaks as shown in the following table:
| 2θ(°) | relative intensity, [(I/I₀)×100] |
|---|---|
| 7.59±0.2 | 100 |
| 10.81±0.1 | 5-50 |
| 16.52±0.1 | 5-50 |
| 17.97±0.1 | 5-50 |
| 23.34±0.05 | 5-50 |
| 34.74±0.05 | 5-50 |

6. The preparation process according to claim 5, **characterized in that** the XRD spectrum of the aluminophosphate precursor further has X-ray diffraction peaks as shown in the following table
| 2θ(°) | relative intensity, [(I/I₀)×100] |
|---|---|
| 14.25±0.1 | 5-50 |
| 21.01±0.1 | 10-20 |
| 24.27±0.05 | 5-50 |
| 26.05±0.05 | 5-50 |
| 27.82±0.05 | 5-50 |
| 28.15±0.02 | 5-50 |
| 30.03±0.02 | 5-50 , |
preferably further has X-ray diffraction peaks as shown in the following table:
| 2θ(°) | relative intensity, [(I/I₀)×100] |
|---|---|
| 12.09±0.1 | 5-50 |
| 19.77±0.1 | 5-50 |
| 31.33±0.01 | 5-50 |
| 38.29±0.01 | 5-50 |

7. The preparation process according to any of claims 4 to 6, **characterized in that** in the synthetic mother liquor, the molar ratio of each material is as follows: silicon source as SiO₂, aluminium source as Al₂O₃, aluminophosphate precursor as Al₂O₃ and P₂O₅, fluorine source as HF, and water as H₂O, (0-0.5)SiO₂:(0.8-5)Al₂O₃:1P₂O₅:(0.01-0.9) quaternary ammonium compound 1:(0.9-5) quaternary ammonium compound 2:(0.1-5)HF:(10-500)H₂O, preferably (0-0.3)SiO₂:(0.85-2)Al₂O₃:1P₂O₅:(0.003-0.85) quaternary ammonium compound 1:(0.95-3) quaternary ammonium compound 2:(0.3-3)HF:(20-300)H₂O, more preferably (0-0.1)SiO₂:(0.0.86-1.5)Al₂O₃:1P₂O₅:(0.05-0.8) quaternary ammonium compound 1:(1-2) quaternary ammonium compound 2:(0.5-1.8)HF:(50-130)H₂O.

8. The preparation process according to any one of claims 4-7, **characterized in that** the quaternary ammonium compound 1 is one or more of tetramethylammonium hydroxide, tetraethylammonium hydroxide, tetrapropylammonium hydroxide and others;
the quaternary ammonium cation in the quaternary ammonium compound 2 is 6-N,N-dimethylaminohexyl-2-hydroxyethyldimethylammonium ion or 6-N,N-dimethylaminohexyl-ethyldimethylammonium ion.

9. The preparation process according to any one of claims 4-8, **characterized in that** the aluminium source is one or more pseudo-boehmite, aluminium isopropoxide, alumina sol, alumina, aluminium chloride, aluminium sulfate, hydrated alumina, sodium metalluminate and aluminium hydroxide;
the silicon source is one or more of silica sol, fumed silica, ethyl orthosilicate, silicic acid and silica gel;
the fluorine source is one or more of hydrofluoric acid or ammonium fluoride.

10. The preparation process according to any one of claims 4-9, **characterized in that** in step b), the crystallization conditions are as follows: in a first stage: the crystallization temperature is 120°C-150°C, the crystallization time is 24-84 hours, preferably, the crystallization temperature is 120°C-140°C, the crystallization time is 48-80 hours; in a second stage: the crystallization temperature is 170°C-200°C, the crystallization time is 2-24 hours, preferably, the crystallization temperature is 180°C-200°C, the crystallization time is 4-18 hours.

11. The preparation process according to any one of claims 4-10, **characterized in that** after step b), a separation step is further included, preferably the separation step comprises at least one of centrifugal separation, washing and drying.

12. A molecular sieve composition, containing the molecular sieve according to any one of claims 1-3 or the molecular sieve prepared with the preparation process according to any one of claims 4-11, and a binder.

13. The molecular sieve composition according to claim 12, wherein, the binder is one or more of nano-silica, alumina, diatomite, and hollow ceramic ball.

14. A catalyst for producing dimethyl ether from methanol, containing the molecular sieve according to any one of claims 1-3, or the molecular sieve prepared with the preparation process according to any one of claims 4-11, or the molecular sieve composition according to claim 12 or 13.

15. A method for producing dimethyl ether from methanol, which comprises a step of converting methanol to dimethyl ether in the presence of a catalyst, wherein the catalyst contains the molecular sieve according to any one of claims 1-3, or the molecular sieve prepared with the preparation process according to any one of claims 4-11, or the molecular sieve composition according to claim 12 or 13.
